# EUROPÄISCHE PATENTANMELDUNG

(11) **EP 0 734 705 A2**
(43) Veröffentlichungstag der Anmeldung: **02.10.1996**
(21) Anmeldenummer: 96250055.9
(22) Anmeldetag: 04.03.1996
(51) Int. Cl.: A61F 5/56

(54) **Vorrichtung zum Verhindern unerwünschten Schnarchens beim Menschen**

(30) Priorität: 03.03.1995 DE 29503604 U
(71) Anmelder: Haggert, Horst-Jürgen, W-64579 Gernsheim (DE)
(72) Erfinder: Haggert, Horst-Jürgen, W-64579 Gernsheim (DE)
(74) Vertreter: Christiansen, Henning, Dipl.-Ing.

(57) **Zusammenfassung**

Vorrichtung zum Verhindern unerwünschten Schnarchens beim Menschen, welche in den Mund/Rachenraum einsetzbar ist und ein Andruckmittel für die Zunge aufweist, mit einem Feuchtereservoir (40) zur Speicherung und dosierten Abgabe einer den Rachenraum vor Austrocknung bewahrenden Flüssigkeit.

## Beschreibung

Die Erfindung betrifft einen Vorrichtung der im Oberbegriff des Anspruchs 1 angegebenen Art.

Das Problem des Schnarchens und die damit verbundenen gesundheitlichen und sozialen Probleme sind seit langem bekannt.

Für Personen, welche im gleichen Raum nächtigen, stellt Schnarchen eine mögliche starke gegenseitige Belästigung dar.

Es sind bereits verschiedenartigste Vorrichtungen zum Verhindern des Schnarchens bekannt geworden, bei denen insbesondere ein unabsichtliches Öffnen des Mundes vermieden oder aber die Position der Kiefer in einer vorbestimmten Stellung arretiert soll. Als Beispiele hierfür seien die DE-U-73 15 382 und die DE-U-94 09 999 genannt.

Darüber hinaus sind Lösungen bekannt, mittels denen insbesondere das Schnarchen in Rückenlage verhindert werden soll, indem durch konstruktive Maßnahmen ein Absinken der Zunge in den Rachenraum verhindert wird. Hierzu wird beispielsweise ein Einnähen bzw. Anheften von Polstern, Kugeln etc. im Rückenteil der Schlafbekleidung vorgeschlagen, welche das Schnarchen des Schlafenden verhindern sollen.

Ebenso ist bereits vorgeschlagen worden, den Kopf einer in Rückenlage schlafenden Person durch Kopfpolster oder Nakkenpolster in eine Seitenposition zu verbringen, um ein Zurückfallen der Zunge auszuschließen. Die gleiche Wirkung soll durch eine Kopfstütze, eine Nackenstütze, eine Bruststütze, eine Halskrause bzw. eine Nackenkrause erreicht werden.

Es ist schließlich auch bekannt, das Schnarchgeräusch mittels eines Sensors, beispielsweise eines Mikrofons, zu erfassen und in geeignete Impulse umzuwandeln, um den Schnarchvorgang während des Schlafes zu unterbinden oder den Schnarcher mittels Vibrationen oder Tönen, beispielsweise durch ein Vibrationskopfkissen, zu wecken.

Eine andere bekannte Lösung schlägt einen Einsatz in die Nasenlöcher vor, durch welchen der Querschnitt der Nasenlöcher der betreffenden Person erweitert wird, um eine bessere Nasenatmung zu ermöglichen.

Alle bekannten Geräte und Vorrichtungen haben mehr oder weniger Mängel in der Weise, daß
- das Schnarchen nicht ganz unterbunden wird,
- die Benutzung lästig ist,
- der Mund entweder geschlossen oder geöffnet sein muß,
- bei geöffneten Mund die Rachenschleimhäute austrocknen, womit die Gefahr einer Erkältung oder Heiserkeit besteht,
- der Anwender in seiner Bewegungsfreiheit beim Schlaf behindert ist und
- die Schlafphase unterbrochen
wird.

Ausgehend von den Mängeln des Standes der Technik liegt der Erfindung die Aufgabe zugrunde, einen Vorrichtung der vorgenannten Gattung zu schaffen, welcher den Anwender in seiner durch die Schlafgewohnheiten bedingten Bewegungsfreiheit nicht einengt, ein Öffnen und Schließen des Mundes zuläßt und demzufolge eine Nasenatmung und/oder Mundatmung ermöglicht.

Diese Aufgabe wird mit den kennzeichnenden Merkmalen des Anspruchs 1 gelöst.

Die Erfindung schließt die Erkenntnis ein, daß das Schnarchen dadurch wirksam verhindert werden kann, daß ein Austrocknen der Rachenschleimhäute während des Schnarchens bei geöffneten Mund unterbunden wird. Auf wirksame Weise kann dies durch eine Vorrichtung geschehen, die innerhalb des Rachenraums ein ständiges Befeuchten der Rachenschleimhäute ermöglicht. Eine derartige Vorrichtung eignet sich in günstiger Weise auch als Träger eines Substrats zur Aufnahme eines Aerosols zum Inhalieren.

Eine Verhinderung des Austrocknens wird durch ein Feuchtigkeitsreservoir erzielt, welches in den Rachenraum eingebracht und dabei an einer Halterung befestigt wird, die sich (elastisch abgefedert) an Zunge und Gaumen abstützt. Auf diese Weise werden einerseits Zunge und Rachenraum in einer Position gehalten, welche ein Entstehen des Schnarchens in einer seiner Ursachen unterbindet, wobei die Feuchtigkeitszufuhr eine entscheidende Vermittlungswirkung bei der Beseitigung dieser Ursache hat.

Entsprechend einer bevorzugten Ausführungform der Erfindung weist der Vorrichtung eine Gaumenmatrix, eine Zungenmatrix und einen Zungen-Rückhalter auf, welche beweglich miteinander verbunden sind. Vorzugsweise sind Gaumenmatrix, Zungenmatrix und Zungen-Rückhalter in einem Mehrfachgelenk, vorzugsweise einem Doppelgelenk um eine gemeinsame Achse schwenkbar zusammengeführt, wobei jedes der Teile relativ zu einem der anderen bewegt werden kann.

An der Gaumenmatrix ist eine in Richtung des Gaumendaches weisende, lösbar befestigte Vorrichtung mit einem Träger vorgesehen, an welchem ein sphärisch, vorzugsweise als Kugelabschnitt, ausgebildetes Kissen aus einem elastischen, medizinisch unbedenklichen Werkstoff angeordnet ist. Dieses Kissen paßt sich in vorteilhafter Weise selbsttätig beim Andrücken an das Gaumendach an die Form des Gaumendaches an und ist für eine Flüssigkeit, beispielsweise eine Emulsion, aufnahmefähig ausgebildet. Bei Zusammendrücken des Kissens tritt jeweils eine Teilmenge der gespeicherten Flüssigkeit aus und hält dem Mund-/Rachenraum feucht. Die Befeuchtung kann dabei durch unmittelbare Abgabe der Flüssigkeit an den benachbarten Gaumen oder aber durch Verdampfung erfolgen.

Die Abstützung erfolgt innerhalb des Rachenraums bevorzugt an drei Punkten, so daß sich eine stabile Fixierung ergibt, welche den Benutzer nicht beeinträchtigt. Die Federsteifigkeiten der die Gelenkverbindung kontrollierenden Federelemente sind dabei bevorzugt insgesamt so gewählt, daß Zungenmatrix und Zungenrückhalter die Basisabstützungen für eine bogenbrückenartige Konstruktion bilden, welche dazu tendiert, ihre gestreckte Lage beizubehalten und sich dabei über das elastische Feuchtereservoir am oberen Gaumenraum weich abstützt. Die veränderbare Krümmung dient dabei zusammen mit der variablen Länge des Zungenrückhalters zur individuellen Anpassung an die anatomischen Gegebenheiten. Eine Stabilisierung der so erreichten Einstellung erfolgt durch die Zungenmatrix, welche bei weicher Auflage auf der Zunge und entsprechend nachgiebieger Auslegung der die relative Position von Zungen- und Gaumenmatrix bestimmender Feder der Kieferbewegung zum Öffnen des Mundes folgen kann. Infolge der sich ergebenden Hebelwirkung erzeugt die Reaktionskraft der Zungenmatrix bei Auflage auf die Zunge wiederum eine Tendenz der zuvor beschriebenen bogenbrückenartigen Konstruktion, sich an die Innenseite des oberen Gaumens anzulegen, so daß das Feuchtereservoir in Position gehalten wird. Auf diese Weise ergibt sich alles in allem eine stabile Fixation der Vorrichtung, trotz geringer Reaktionskräfte im Auflagebereich und damit nur unwesentliche Beeinträchtigung des Benutzers.

Nach einer vorteilhaften Weiterbildung der Erfindung sind für die lösbare Befestigung der als Rachenbefeuchter konzipierten Vorrichtung an dem Träger Haltemittel vorgesehen, welche eine in der Gaumenmatrix angeordnete Ausnehmung hintergreifen. Entsprechend einer anderen günstigen Weiterbildung der Erfindung besteht das Kissen aus einem flexiblen Kunststoffschaummaterial. Das Reservoir ist insbesondere so ausgebildet, daß es einen Flüssigkeitsvorrat aufnehmen und über die Schlafphase, d.h. mehrere Stunden dosiert kontinuierlich in der Weise abgibt, daß der Rachenraum befeuchtet wird. Dieser Vorgang wird unterstützt durch die am Reservoir vorbeistreichende Atemluft.

Zum Anschluß des Zungen-Rückhalters an das Doppelgelenk ist ein Zungenhalter-Verbindungstück vorgesehen, welches an einem Ende als Gelenkteil ausgebildet ist und an dem anderen Ende eine Aufnahmevorrichtung trägt, in die der Zungen-Rückhalter einsteckbar ist und durch Rastmittel in der gewählten Position gehalten wird. Dadurch können in Anpassung an die Größe des Mund- und Rachenraumes auf einfache Weise unterschiedlich lange Zungen-Rückhalter in das Verbindungsstück für den Zungen-Rückhalter eingesetzt werden.

Die Gaumenmatrix trägt gelenkseitig eine Verstrebung, welche mit ihrer Länge den Abstand der Gaumenmatrix zur Zungenmatrix und dem Verbindungsstück für den Zungen-Rückhalter bestimmt.

Nach der bevorzugten Ausführungsform der Erfindung ist an der dem Gaumen abgewandten Seite der Gaumenmatrix eine Vorrichtung zur Aufnahme von Inhalationsstoffen angeordnet. Diese Vorrichtung ist fest mit der Gaumenmatrix verbunden und beispielsweise als Hohlzylinder mit einer Durchbrüche aufweisenden Mantelfläche ausgebildet

Entsprechend einer anderen bevorzugten Ausführungsform der Erfindung ist die die Gaumenmatrix mit dem Doppelgelenk verbindende Verstrebung zwecks Vereinfachung der Konstruktion des Vorrichtunges als Vorrichtung zur Aufnahme von Inhalationsstoffen ausgebildet.

Diese Verstrebung sichert auf einfache Weise, daß bei Bewegungen des Unterkiefer und oder Schließen des Mundes die Distanz zwischen Gaumenmatrix, Zungenmatrix und Zungen-Rückhalter bzw. Verbindungsstück für den Zungen-Rückhalter an dem als Doppelgelenk ausgebildeten Verknüpfungspunkt erhalten bleibt.

Lediglich in Richtung des schlundseitigen Ausgangs des Rachenraumes ist der Abstand zwischen Gaumenmatrix und Zungenmatrix veränderbar. Dadurch ist bei Benutzung der erfindungsgemäßen Vorrichtung in günstiger Weise eine Nasenatmung und/oder eine Mundatmung möglich.

Die Veränderung der Distanz zwischen der Gaumenmatrix und der Zungenmatrix ist durch ein erstes Federelement steuerbar, welches an den einander zugewandten Innenseiten der Gaumenmatrix bzw. der Zungenmatrix angeordnet und mit diesen fest verbunden ist. Entsprechend der bevorzugten Ausführungsform der Erfindung ist das erste Federelement als eine auf Torsion belastete Spiralfeder, beispielsweise als Schenkelfeder, ausgebildet.

Durch die Wirkung des ersten Federelements wird die oberhalb der Gaumenmatrix befindliche Vorrichtung zum Befeuchten des Rachenraums ständig an das Gaumendach gedrückt und der erfindungsgemäßen Vorrichtung kann während dem Schlaf nicht mit der Zunge aus dem Rachenraum bewegt werden.

Der Zungen-Rückhalter ist entsprechend einer vorteilhaften Weiterbildung der Erfindung T-förmig ausgebildet, wobei die kurzen freien Enden des T aus der Ebene des langen , vorzugsweise laschenförmig ausgebildeten freien Endes des T im wesentlichen bogenförmig herausgeführt sind. Dadurch kann sich der Zungen-Rückhalter vollflächig an die Zungenoberfläche anlegen. Die Länge des in die Aufnahmevorrichtung des Verbindungsstück für den Zungen-Rückhalters einsteckbaren T-förmigen Zungen-Rückhalters ist durch Änderung der Laschenlänge variabel, so daß der Zungen-Rückhalter auf einfache Weise an die anatomischen Bedingungen des Rachenbereiches anpaßbar ist. Dadurch kann die in den Rachenraum hineinragende Länge des Zungen-Rückhalter eingestellt werden, um zu verhindern, daß bei zu weitem Hineinragen des Zungen-Rückhalters ein Brechreiz ausgelöst wird. Zungen-Rückhalter und Verbindungsstück für den Zungen-Rückhalter bilden nach dem Zusammenstecken zwar eine bauliche Einheit, aber der Zungen-Rückhalter kann vom Zungen-Rückhalter-Verbindungstück gelöst und gegen einen kürzeren oder längeren Zungen-Rückhalter ausgetauscht werden. Dazu weist das Verbindungsstück für den Zungen-Rückhalter an seinem dem Doppelgelenk abgewandten Ende eine Aufnahmevorrichtung auf, welche zur axialen Sicherung der Position des Zungen-Rückhalters mit einem Rastmittel versehen ist.

Zur Steuerung der Schwenkbewegung des Zungen-Rückhalters um den Doppelgelenkpunkt ist ein zweites Federelement vorgesehen. Dieses Federelement verbindet die an der Gaumenmatrix vorhandene Verstrebung mit der Aufnahmevorrichtung des Verbindungsstück für den Zungen-Rückhalters und weist eine größere Federkonstante auf als das erste Federelement, welche Gaumen- und Zungenmatrix koppelt. Dadurch wird die Zunge zurückgehalten, ohne ihre Beweglichkeit vollständig aufzuheben.

Durch die Verbindung in einem Doppelgelenk an dem Verknüpfungspunkt bzw. und die Kopplung mittels des ersten und zweiten Federelements sind die die Verstrebung aufweisende Gaumenmatrix, die Zungenmatrix und das Verbindungsstück für den Zungen-Rückhalter in vorteilhafter Weise unabhängig voneinander bewegbar, wobei die Zunge gleichzeitig ständig angedrückt bleibt.

Entsprechend einer zusätzlichen Weiterbildung der Erfindung weisen die nicht in dem Doppelgelenk verbundenen Enden der Gaumenmatrix und der Zungenmatrix jeweils ein Mittel auf, durch welches in günstiger Weise verhindert wird, daß die erfindungsgemäße Vorrichtung bei Benutzung unkontrolliert in den Rachenraum abgleitet. Diese Mittel sind als Winkelstücke ausgebildet, deren freie Enden sich unter einem Winkel von vorzugsweise 90° in Richtung Nase bzw. Kinn erstrecken. Die abgewinkelten Enden werden zwischen Oberlippe und den Zähnen der Oberkiefers bzw. Unterlippe und den Zähnen des Unterkiefers positioniert, wodurch der Vorrichtung an der vorgesehenen Position im Mundraum gehalten wird und durch zwangsläufige Bewegungen der Kiefer nicht ungewollt in den Rachenraum gleiten kann.

Für die einzelnen Elemente des erfindungsgemäßen Vorrichtunges ist als medizinisch unbedenklicher Werkstoff vorzugsweise ein Polyacrylat vorgesehen.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: eine bevorzugte Ausführungsform der Erfindung in Seitenansicht,
- Figur 2: die in Figur 1 gezeigten Ausführungsform in Draufsicht,
- Figur 3: die Darstellung des Details Z gemäß Figur 1,
- Figuren 4a bis 4c: die gesonderte Darstellung einer in den Figuren 1 und 2 gezeigten Einzelheit,
- Figur 4d: des Details X gemäß Figur 1, sowie
- Figuren 5a bis 5d: die Darstellung der bevorzugten Ausführungsform und einer günstigen Weiterbildung der in den Figuren 1 und 2 gezeigten Gaumenmatrix.

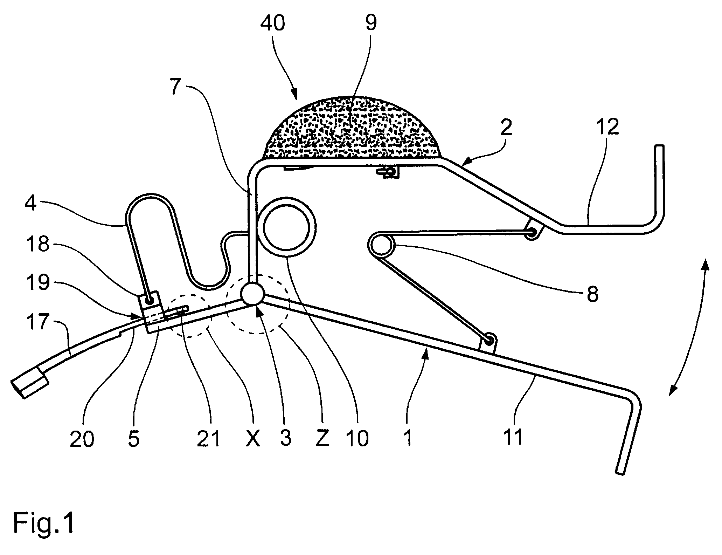

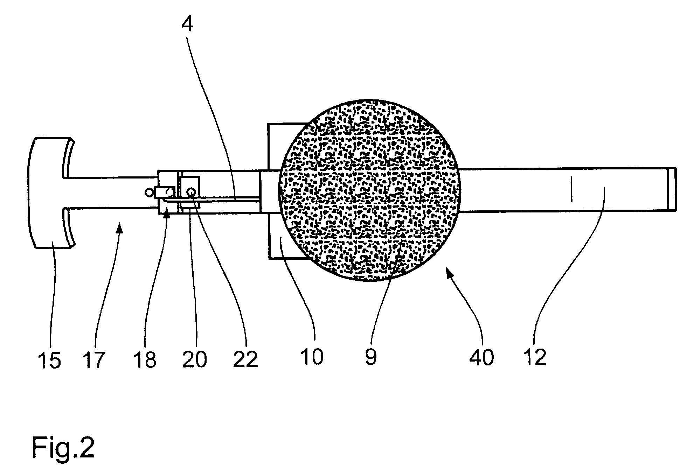

Der in den Figuren 1 und 2 in Seitenansicht und in Draufsicht dargestellten Vorrichtung 100 ist als Einsatz in den Mundraum konzipiert und weist eine Zungenmatrix 1 und eine Gaumenmatrix 2 auf, welche sich auf der Zungenoberfläche bzw. am Gaumenbogen abstützen, wenn die Vorrichtung 100 im Mundraum eines Menschen positioniert wird.

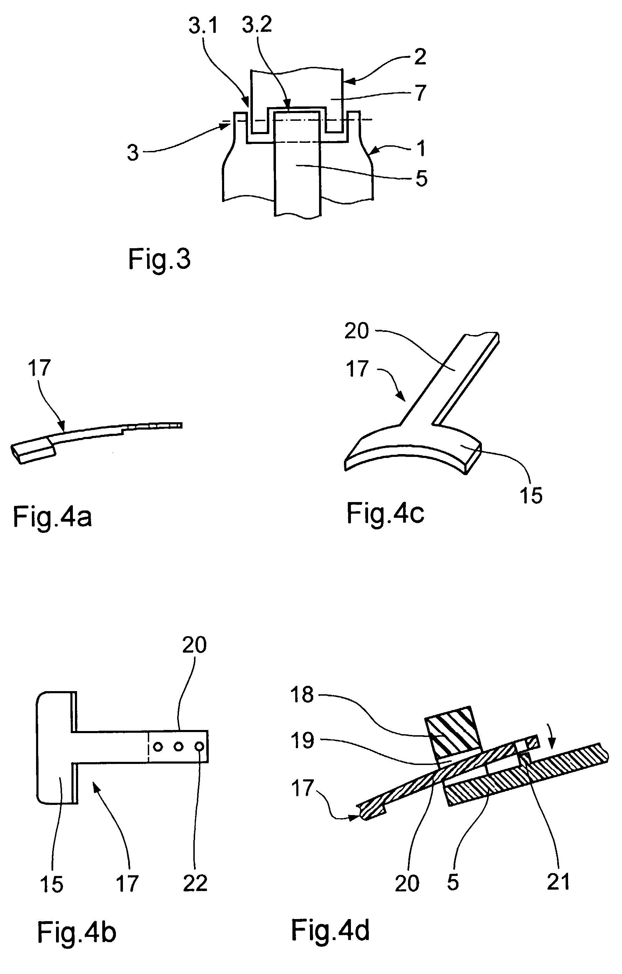

Die Zungenmatrix 1 und die Gaumenmatrix 2 sind in einem Gelenk 3 schwenkbar miteinander verbunden. In dem als Doppelgelenk (vergleiche die Positionen 3.1 und 3.2 in Figur 3) ausgebildeten Gelenk 3 ist gleichzeitig und um dieselbe, horizontal und quer gerichtete Drehachse ein Zungen-Rückhalter 17 über ein Verbindungsstück für den Zungen-Rückhalter 5 schwenkbar gelagert. Die in dem Doppelgelenk 3 miteinander verbundenen Elemente 1, 2, 5 der Vorrichtung 100 sind dadurch unabhängig voneinander bewegbar, wobei die Zunge durch den Zungen-Rückhalter 17 gleichzeitig angedrückt bleibt.

Das Ausmaß der Beweglichkeit der Elemente 1, 2, 5 wird durch ein erstes und eine zweites Federelement 4 und 8 kontrolliert, welche einerseits die Zungenmatrix 1 mit der Gaumenmatrix 2 jeweils an den Matrixinnenseiten in Mittenposition und andererseits das Verbindungsstück für den Zungen-Rückhalter 5 mit einer an der Gaumenmatrix 2 vorgesehenen Verstrebung 7 verbinden.

Die Federelemente 4 und 8 unterscheiden sich entsprechend ihrer Funktion durch ihre Federkonstante und durch ihre Form.

Das die Zungenmatrix 1 mit der Gaumenmatrix 2 koppelnde Federelement 8 ist als auf Torsion belastbare Schraubenfeder ausgebildet, deren relativ langschenklige freien Enden mit Zungen- bzw. Gaumenmatrix verbunden sind. Die Federkonstante dieser Schenkelfeder ist relativ klein und weist einen geringeren Wert auf als die Federkonstante des Federelements 4. Dadurch können die Zungenmatrix 1 und die Gaumenmatrix 2 ohne besonderen Kraftaufwand gegeneinander geschwenkt werden und gestatten problemlos eine Kieferbewegung der die Vorrichtung benutzenden Person, wobei die Zunge gleichzeitig niedergedrückt wird. Durch die Wirkung des Federelements 8 wird die Gaumenmatrix 2 dabei ständig leicht an das Gaumendach gedrückt. Die Beweglichkeit der Vorrichtung unter Vorspannung durch das Federelement 8 ist durch den Doppelpfeil angedeutet.

Das Federelement 4 stellt einen mehrere, in einer Ebene angeordnete Bögen aufweisenden mäanderförmig geformten Federdraht mit einer relativ großen Steifigkeit dar. Dadurch kann der fixierend auf die Zunge gedrückte Zungen-Rückhalter 17 nur durch eine relativ große Kraft gegen die Wirkung des Federelements 4 bewegt werden. In der Zeichnung ist die Feder zur Verdeutlichung in senkrechter Richtung wiedergegeben. Bei einer bevorzugten praktischen Ausführung verlaufen die Mäanderwindungen jedoch in horizontaler Richtung, da sie auf diese Weise der Form des Zungenrückhalters folgen und insoweit nur wenig zusätzlichen Platz im Rachenraum einnehmen.

Die Gaumenmatrix der Vorrichtung trägt auf der dem Gaumen zugewandten Seite eine als Rachenbefeuchter ausgebildete, auswechselbar angeordnete Vorrichtung 40. Die Vorrichtung 40 weist ein elastisches Kissen 9 in Form einer Kugelkappe auf, welches aus einem medizinisch unbedenklichen Werkstoff, vorzugsweise aus einem, vorzugsweise flexiblen, Kunststoffschaum besteht und in der Lage ist, sich der Form des Gaumenbogens anzupassen und eine bestimmte Flüssigkeitsmenge temporär zu speichern. Durch die Kieferbewegung wird das Kissen geringfügig komprimiert, so daß Flüssigkeitsteile freigesetzt werden und den Rachenraum ständig feucht halten. Die Abgabe der Flüssigkeit kann dabei in der Flüssigphase oder durch Verdampfen erfolgen, wodurch eine Benetzung des Rachenraums sichergestellt ist. Durch eine Beigabe von Geschmacks- oder Geruchsstoffen in die freizusetzende Flüssigkeit kann eine weitere Annehmlichkeit bei der Benutzung hervorgerufen werden.

Durch eine zwischen dem Doppelgelenk 3 und der Gaumenmatrix 2 vorgesehene Verstrebung 7 ist unabhängig von der Kieferstellung ein mittlerer Mindestabstand von 1 cm zwischen Zungenmatrix 1 und Gaumenmatrix 2 erreichbar und sichert, daß der Benutzer sowohl in der Nasen- als auch in der Mundatmung nicht beeinträchtigt wird.

Die Vorrichtung 100 weist zusätzlich an der Verstrebung 7 ein weiteres Reservoir 10 zur Aufnahme und temporären Speicherung eines Wirkstoffes auf, mit welchem eine medizinische Behandlung des Mund-/Rachenraumes vorgenommen werden kann. Das Reservoir 10 ist als zylindrischer Hohlkörper mit unverschlossenem Boden- und Deckelbereich ausgebildet. In der Mantelfläche sind eine Vielzahl von Durchbrüchen vorgesehen, so daß der Wirkstoff ohne besondere Schwierigkeiten in den Mund-/Rachenraum austreten kann. Durch den mit der Vorrichtung 10 ausgestatteten Vorrichtung 100 lassen sich beispielsweise Erkältungskrankheiten mit in dem Hohlraum der Vorrichtung 10 deponierten, mentholhaltigen Präparaten medizinisch behandeln.

An dem gelenkfernen Ende des Zungen-Rückhalter-Verbindungsstücks 5 ist eine Aufnahmevorrichtung 18 vorgesehen. Diese weist einen rechteckförmigen Durchbruch 19 auf, durch welchen der Zungen-Rückhalter 17 geschoben und durch ein noppenförmiges, in eine passende Ausnehmung 22 im Zungen-Rückhalter 17 eingreifendes Rastmittel 21 axial gesichert wird.

Der Zungen-Rückhalter 5 ist T-förmig ausgebildet, wobei das laschenförmig ausgebildete, längere freie Ende des T in seiner Länge variiert wird, um eine individuelle Anpassung der Vorrichtung an die jeweiligen Bedingungen des Mund/Rachenraumes vornehmen zu können. Damit kann ein durch einen zu weit in den Rachenraum ragenden Zungen-Rückhalter ausgelöster Brechreiz vermieden werden.

An den gelenkfernen Enden der Zungenmatrix 1 und der Gaumenmatrix 2 sind winkelförmige Mittel 11 und 12 vorgesehen, deren freie Ende sich jeweils - bezogen auf die Längsachse der Zungenmatrix 1 bzw. der Gaumenmatrix 2 - unter einem Winkel von etwa 90° in Richtung des Kinns bzw. in Richtung der Nase erstrecken. Die abgewinkelten Enden werden zwischen den Frontzähnen des Oberkiefers und der Oberlippe bzw. den Frontzähnen des Unterkiefers und der Unterlippe der den Vorrichtung tragenden Person positioniert, so daß ein ungewolltes Zurückgleiten der Vorrichtung aus dem Mundin den Rachenraum verhindert wird. Im Zusammenwirken mit der Vorrichtung 40 zur Rachenbefeuchtung, welche durch die Schraubenfeder 8 stets in die Wölbung des Gaumendaches gedrückt wird, ist dadurch eine ausreichende Lagesicherung der Vorrichtung 100 im Rachenraum gegeben, so daß dieser auch bei Bewegungen während des Schlafs oder durch die Zunge nicht aus dem Mundraum gedrückt werden kann.

Die Figur 3 zeigt als Einzelheit Z das Doppelgelenk 3 in Ansicht von der Seite. In dem Doppelgelenk sind die Gelenkverbindung 3.1 zwischen der Zungenmatrix 1 und der Gaumenmatrix 2 sowie die Gelenkverbindung 3.2 zwischen der Gaumenmatrix 2 und dem Verbindungsstück für den Zungen-Rückhalter 5 auf gleicher Achse liegend zusammengefaßt. Die Verbindung der Gaumenmatrix 2 mit dem Doppelgelenk 3 erfolgt jeweils über die Verstrebung 7. Diese konstruktive Lösung gestattet voneinander unabhängige Schwenkbewegungen der miteinander verbundenen Elemente 1, 2, 5 der Vorrichtung.

In den Figuren 4a bis 4c ist ein Zungen-Rückhalter 17 detailiert dargestellt. Die Figur 4d zeigt die Einzelheit X entsprechend Figur 1. Der T-förmig ausgebildete Zungen-Rückhalter 17 weist zwei kurze freie Enden 15 und ein laschenförmiges, längeres freies Ende 20 auf, wobei die beiden kurzen freien Enden 15 aus der Ebene, in welcher sich das laschenförmige Ende 20 erstreckt, bogenförmig in Richtung des Zungenrückens - bezogen auf die Gebrauchslage - herausgeführt sind. Dadurch paßt sich der Zungen-Rückhalter optimal an die konvexe Form des Zungenrückens an. Die jeweils entsprechend der erforderlichen wirksamen Länge des Zungen-Rückhalters 17 gewählte kreisförmige Ausnehmung 22 in dem laschenförmigen freien Ende 20 wird beim Verbinden des Zungen-Rückhalters 17 mit dem Verbindungsstück für den Zungen-Rückhalter mit dem noppenförmigen Rastmittel 21 in Wirkungseingriff gebracht, nachdem der Zungen-Rückhalter durch die Ausnehmung 19 der Aufnahmevorrichtung 18 des Verbindungsstück für den Zungen-Rückhalters 5 geschoben worden ist. Dies ermöglicht die Sicherung des mittels einer Steckverbindung positionierten Zungen-Rückhalters 17 gegen ein axiales Verschieben. Der entsprechende Bewegungsablauf ist durch die Richtung des Pfeils in Figur 4d angedeutet.

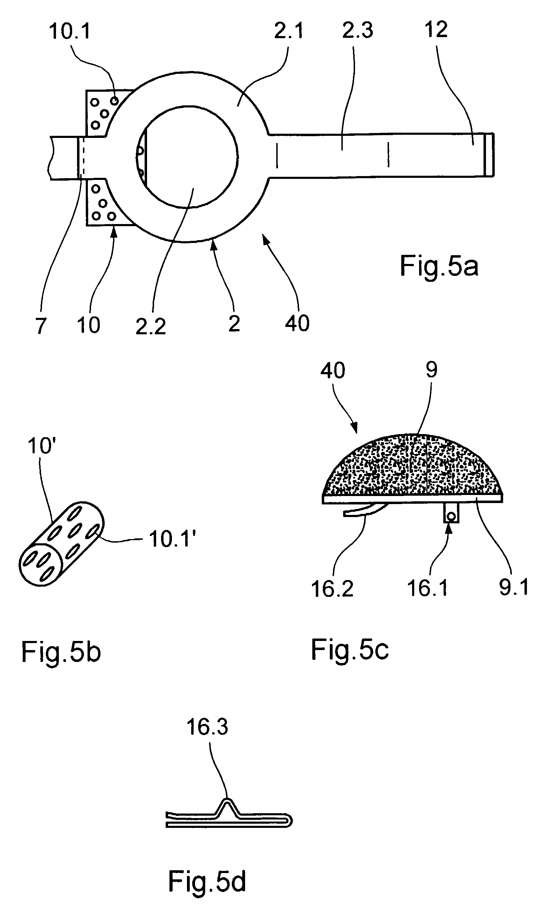

In den Figuren 5a bis 5d sind die Elemente der Gaumenmatrix 2 im einzelnen dargestellt.

Figur 5a zeigt dabei die Gaumenmatrix 2 in Draufsicht bei herausgelöstem Rachenbefeuchter 40. Die Gaumenmatrix 2 weist einen kreisringförmigen Mittelabschnitt 2.1 auf, an welchen zum einen, die in der Zeichnungsebene nach hinten weisende Verstrebung 7 und zum anderen ein Steg 2.3 angeformt sind. Das freie Ende 12 des Steges 2.3 ist abgewinkelt ausgebildet und schließt einen Winkel von etwa 90° ein. An die sich unterhalb des Mittelabschnitts 2.1 der Gaumenmatrix 2 befindliche Verstrebung 7 ist die Vorrichtung 10 angeformt, welche zur Aufnahme und temporären Speicherung eines (nicht dargestellten) medizinischen Wirkstoffes zur Behandlung von Erkrankungen des Mund- oder Rachenraumes vorgesehen ist. Die Vorrichtung 10 ist als zylindrischer Hohlkörper mit offener Boden- und Deckfläche ausgebildet. Die im Mantel des Hohlkörpers vorgesehenen Ausnehmungen 10.1 sind kreisförmig ausgebildet und ermöglichen den Durchtritt eines beispielsweise in pastöser Form eingebrachten Wirkstoffes durch die Hohlkörperwandung in den Mund- und/oder Rachenraum.

In Figur 5b ist eine Weiterbildung der Erfindung mit einer die Vorrichtung 10' dargestellt, welche in ihrer Mantelfläche langlochförmige Ausnehmungen 10.1' aufweist.

In die Ausnehmung 2.2 des kreisringförmigen Mittelabschnitts 2.1 ist die in Figur 5c dargestellte Vorrichtung 40 einsetzbar. Die Vorrichtung 40 ist als Rachenbefeuchter ausgebildet und weist eine Trägerplatte 9.1 auf, an welcher eine Kappe 9 in Form eines Kugelabschnitts befestigt ist. Die Kappe 9 besteht aus einem elastischen, medizinisch unbedenklichen Kunststoffschaum. Der Kunststoffschaum ist in der Lage eine bestimmte Flüssigkeitsmenge aufzunehmen und mindestens temporär zu speichern. Bei einer Kieferbewegung wird die Kappe 9 (u.a. durch die Wirkung des Federelements 8 entsprechend Figur 1) gegen die Gaumenwandung gedrückt, so daß ein Teil der in der Kappe 9 gespeicherten Flüssigkeit in den Mund-/Rachenraum austreten und diesen befeuchten kann.

An der Unterseite der Trägerplatte 9.1 sind Haltemittel 16.1, 16.2 und 16.3 vorgesehen, welche die in der Gaumenmatrix 2 befindliche Ausnehmung 2.2 hintergreifen, wenn die Vorrichtung 40 an der Gaumenmatrix befestigt worden ist. Die Haltemittel sind als eine Bohrung aufweisender Zapfen 16.1, als Haken 16.2 und als doppelschenklige, federnde Klammer 16.3 ausgebildet. Bei befestigter Vorrichtung 40 befindet sich der Kreisring 2.1 der Gaumenmatrix 2 in dem Freiraum zwischen der Trägerplatte 9.1 und dem Haken 16.2. Diese Position wird durch die Klammer 16.3 gesichert, welche sich - mit einem Schenkel durch die Bohrung in dem Zapfen 16.1 geschoben - an der Unterseite der Trägerplatte 9.1 abstützt.

Die Erfindung beschränkt sich in Ihrer Ausführung nicht auf das vorstehend angegebene Ausführungsbeispiel. Vielmehr ist eine Anzahl von Varianten denkbar, welche von der dargestellten Lösung auch bei grundsätzlich anders gearteten Ausführungen Gebrauch macht.

## Patentansprüche

1. Vorrichtung zum Verhindern unerwünschten Schnarchens beim Menschen, welche in den Mund/Rachenraum einsetzbar ist und ein Andruckmittel für die Zunge aufweist,
**gekennzeichnet durch,**
ein erstes Reservoir (40) zur Speicherung und dosierten Abgabe einer den Rachenraum befeuchtenden und somit vor Austrocknung bewahrenden Flüssigkeit.

2. Vorrichtung nach Anspruch 1, **gekennzeichnet durch** eine zur Abstützung auf der Zunge vorgesehene Zungenmatrix (1), deren rückwärtige, insbesondere um eine horizontale Achse (3) schwenkbar verbundene, Verlängerung einen Zungen-Rückhalter (17) bildet.

3. Vorrichtung nach einem der vorhergehenden Ansprüche, **dadurch gekennzeichnet**, daß eine zur Anlage an dem Gaumen ausgestaltete Gaumenmatrix (2) vorgesehen ist, welche mit der Zungenmatrix, um eine horizontale Achse (3) schwenkbar verbunden ist, wobei insbesondere die schwenkbare Verbindung des Zungen-Rückhalters und der Gaumenmatrix um eine gemeinsame Achse, vorzugsweise mittels eines Doppel- oder Mehrfachgelenks (3, 3.1, 3.2), schwenkbar gelagert sind.

4. Vorrichtung nach einem der vorangehenden Ansprüche, **dadurch gekennzeichnet,** daß der Zungen-Rückhalter (17), insbesondere durch ein entsprechendes Verbindungsstück, mit dem Gelenk, (3, 3.1, 3.2) verbunden ist, welches eine Aufnahmevorichtung (18) mit einem vorzugsweise rechteckförmigen Durchbruch (19) und einem, vorzugsweise noppenförmigen, Rastmittel (21) zum Arretieren des einsteckbaren Zungen-Rückhalters (17) aufweist und/oder daß der Zungen-Rückhalter (17) im wesentlichen in Querrichtung T-förmig ausgebildet ist, und die freien Enden (15) des Querbalkens des "T" bogenförmig in Richtung der Zunge weisen.

5. Vorrichtung nach einem der vorangehenden Ansprüche, **dadurch gekennzeichnet,** daß zur Kontrolle der Relativbewegung zwischen Zungenmatrix (1) und Gaumenmatrix (2) ein erstes Federelement (8) und/oder zwischen der Gaumenmatrix (2) und dem Zungen-Rückhalter (5) ein zweites Federelement(4) vorgesehen ist, wobei die Federelemente insbesondere jeweils entweder eine Feder bilden, deren wenige Windungen aufweisende Schraubenfedern (8) nach Art der Feder einer Schenkelfeder radial gerichtete freie Enden aufweisen oder eine Feder mit Mäanderform (4) bilden, an deren Enden jeweils der Kraftangriff erfolgt.

6. Vorrichtung nach einem der vorangehenden Ansprüche, **dadurch gekennzeichnet,** daß das erste Reservoir (9) aus einem saugfähigen Kissen (9), insbesondere aus einem elastischen Werkstoff, besteht und/oder an der dem Gaumendach zugewandten Seite der Gaumenmatrix (2) auswechselbar angeordnet ist.

7. Vorrichtung nach einem der vorangehenden Ansprüche, **dadurch gekennzeichnet,** daß das erste Reservoir (9) in seinem dem Gaumen zugewandten Bereich sphärisch konvex ausgebildet ist.

8. Vorrichtung nach einem der vorangehenden Ansprüche, **dadurch gekennzeichnet,** daß ein lösbares Trägerelement (9.1) für das erste Reservoir Haltemittel (16.1, 16.2, 16.3) zum Hintergreifen einer in der Gaumenmatrix (2) vorgesehen Ausnehmung (2.2) aufweist.

9. Vorrichtung nach einem der vorhergehenden Ansprüche, **gekennzeichnet durch** ein weiteres Reservoir (10) zur Speicherung mindestens eines flüssigen oder insbesondere auch pastösen Medikaments, vorzugsweise eines Inhalationsstoffes, zur Behandlung des Rachenraumes.

10. Vorrichtung nach Anspruch 9, **dadurch gekennzeichnet,** daß das weitere Reservoir (10) aus einem an der dem Gaumen abgewandten Seite der Gaumenmatrix (2) befestigten Aufnahmeraum, insbesondere in Form eines Hohlzylinders, besteht, dessen Mantelfläche eine Mehrzahl von, vorzugsweise kreisförmigen, Durchbrüchen als Austrittsöffnungen (10.1, 10.1') für das Medikament aufweist.

11. Vorrichtung nach einem der vorhergehenden Ansprüche, **dadurch gekennzeichnet,** daß die Gaumenmatrix (2) in eine erste Abwinklung in Form eines Winkelstücks übergeht, welche im Bereich ihres freien Endes die oberen Frontzähne bzw. den Gaumen in Richtung nach außen umgreift und/oder daß die Zungenmatrix in eine zweite Abwinklung in Form eines Winkelstücks übergeht, welche im Bereich ihres freien Endes die unteren Frontzähne bzw. den Gaumen in Richtung nach außen umgreift, um ein Zurückgleiten in den Rachenraum zu verhindern, wobei die Abwinklungen bevorzugt in einer im wesentlichen senkrechten Richtung enden.

12. Vorrichtung nach einem der vorhergehenden Ansprüche, **dadurch gekennzeichnet,** daß als Werkstoff für die Zungenmatrix (1), die Gaumenmatrix (2), den Zungen-Rückhalter (17) und/oder das Verbindungsstück für den Zungen-Rückhalter (5) ein Polyacrylat vorgesehen ist.
